# EUROPEAN PATENT APPLICATION

(11) **EP 0 989 110 A1**
(43) Date of publication of application: **29.03.2000**
(21) Application number: 98911173.7
(22) Date of filing: 01.04.1998
(51) Int. Cl.: C07C 69/675, C07C 67/00, C07F 7/18

(54) **OPTICALLY ACTIVE $g(b)-HYDROXY-$g(a)-BROMO-$g(a)-FLUORO CARBOXYLIC ACID ESTER, PROCESS FOR CATALYTIC ASYMMETRIC SYNTHESIS THEREOF, 2-BROMO-2-FLUOROKETENE SILYL ACETAL, AND PROCESS FOR PRODUCING THE SAME**

(30) Priority: 14.05.1997 JP 12429897
(71) Applicant: Daikin Industries, Limited, Osaka-shi, Osaka 530-0015 (JP)
(72) Inventor: ISEKI, Katsuhiko MEC Laboratory,, Tsukuba-shi Ibaraki 305-0841 (JP); KUROKI, Yoshichika MEC Laboratory,, Tsukuba-shi Ibaraki 305-0841 (JP); KOBAYASHI, Yoshiro, Tokyo 150-0042 (JP)
(74) Representative: ter Meer, Nicolaus, Dipl.-Chem., Dr.
(86) International application number: JP9801521
(87) International publication number: WO9851658

(57) **Abstract**

To provide new optically-active β-hydroxy-α-bromo-α-fluorocarboxylates which are expected to be useful as synthetic intermediates of physiologically-active substances and a process for catalytic preparation of these optically-active substances in high asymmetric yield, and further to provide new 2-bromo-2-fluoroketenesilylacetals which are also useful as synthetic intermediates of these optically-active substances and a synthetic process thereof.

New optically-active β-hydroxy-α-bromo-α-fluorocarboxylates are prepared by catalytic asymmetric synthesis wherein new 2-bromo-2-fluoroketenesilylacetals, which are obtained by reacting dibromofluoroacetates with halogenated silyl compounds, are reacted with aldehydes in the presence of a catalytic amount of chiral Lewis acid.

## Description

### APPLICABLE INDUSTRIAL FIELD OF THE INVENTION

The present invention relates to optically-active β-hydroxy-α-bromo-α-fluoro-carboxylates and a process for catalytic asymmetric synthesis thereof , and to 2-bromo-2-fluoroketenesilylacetals and a preparation process thereof.

### PRIOR ART

Optically-active fluorine-containing compounds have attracted considerable attention in medicinal and agricultural chemical areas in recent years because their fluorine atoms are expected to show new physiological activity.

Recently, the present inventors have reported a catalytic synthetic process of β-hydroxy-α,α-difluorocarboxylates which are optically-active fluorine-containing compounds (Iseki K. ; Kuroki Y. ; Asada D. ; Kobayashi Y., Tetrahedron Lett. 38, 1447, (1997)).

Preparation of 2, 2-difluoroketenesilylacetals herein used as synthetic materials has been studied by several research groups. For example, Kobayashi et al. reported that the acetals were prepared by treating organozinc complex (Reformatsky reagent) which was obtained by reacting methyl iododifluoroacetate with zinc powder in acetonitrile with trialkylsilyl chlorides (Japanese Patent No. 0267250 and Tetrahedron Lett. 29, 1803, (1988)). In addition, Weigel et al. reported that after treating chlorotrimethyl silane and phenyl chlorodifluorothioacetate with zinc powder, the structure of the objective product obtained was confirmed by ¹⁹F-NMR spectrum (Japanese patent opening No. 7-285973).

### OBJECTS OF THE INVENTION

The objects of the present invention are to provide new optically-active β-hydroxy-α-bromo-α-fluorocarboxylates which are expected to be useful as synthetic intermediates of physiologically-active substances and a catalytic synthetic process thereof in high asymmetric yields, and to provide new 2-bromo-2-fluoroketenesilylacetals which are also useful as synthetic intermediates of the foregoing optically-active carboxylates and a synthetic process thereof.

### CONSTITUTION OF THE INVENTION

According to the present invention, the first invention relates to optically-active β-hydroxy-α-bromo-α-fluorocarboxylates represented by the following General Formula [I] or [II] which are expected to be useful as synthetic intermediates of physiologically-active substances. 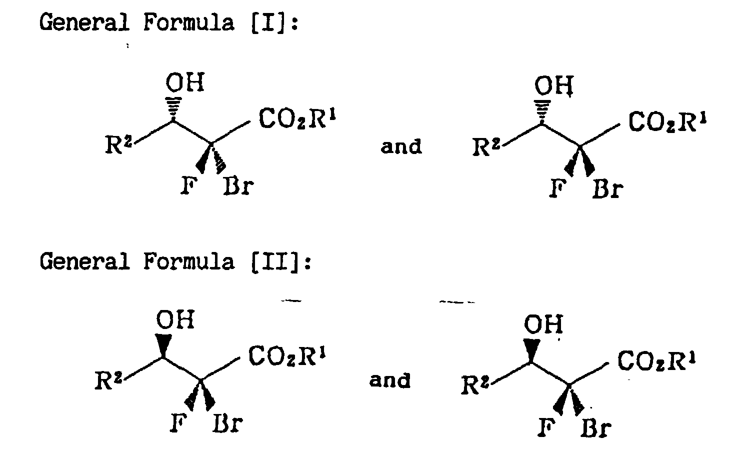 (wherein R's of General Formulas [I] and [II] above are alkyl, aryl, aryloxyaryl, alkyloxyalkyl, alkyloxyaryl or aryloxyalkyl groups; R²s are alkyl, aryl, alkoxy, aryloxy, aryloxyaryl, alkyloxyalkyl, alkyloxyaryl or aryloxyalkyl groups; and R¹ and R² may be either the same or different groups to each other.)

As part of the present invention, the second invention relates to a process for catalytic asymmetric syntheses of the optically-active β-hydroxy-α-bromo-α-fluorocarboxylates as described previously in the first invention. Namely, the catalytic asymmetric syntheses of the optically-active carboboxylates represented by the following General Formula [I] or [II] are performed by reacting 2-bromo-2-fluoroketenesilylacetals represented by the following General Formula [III] with aldehydes represented by the following General Formula [IV] in the presence of a catalytic amount of chiral Lewis acid represented by the following General Formula [V].

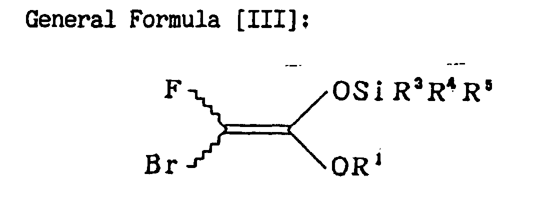

R²CHO General Formula [IV]

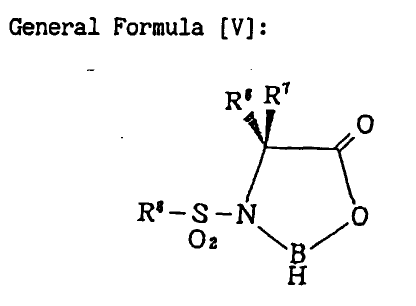

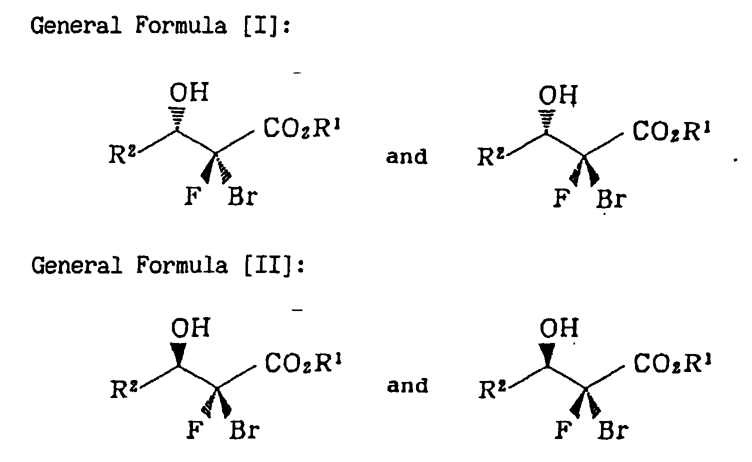

[wherein R¹s of General Formulas [I], [II], [III], [IV] and [V] above are alkyl, aryloxy, aryloxyaryl, alkyloxyalkyl, alkyloxyaryl or aryloxyalkyl groups; R², R³, R⁴, R⁵, R⁶, R⁷ and R⁸ are alkyl, aryl, alkoxy, aryloxy, aryloxyaryl, alkyloxyalkyl, alkyloxyaryl or aryloxyalkyl groups; R¹, R², R³, R⁴, R⁵, R⁶, R⁷ and R⁸ may be either the same or different groups to each other (herein, R⁶ and R⁷ are not the same as each other and either R⁶ or R⁷ may be a hydrogen atom).] As another part of the present invention, the third invention relates to 2-bromo-2-fluoroketenesilylacetals represented by the following General Formula [III], which are useful as reactants (starting materials) of the above second invention. 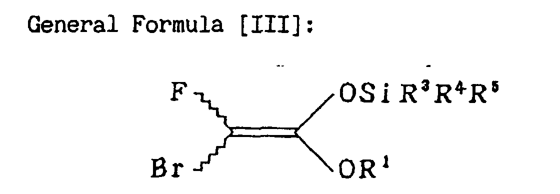 (where R¹ of General Formula [III] above is alkyl, aryl, aryloxyaryl, alkyloxyalkyl, alkyloxyaryl or aryloxyalkyl groups; R³, R⁴ and R⁵ are alkyl, aryl, alkoxy, aryloxy, aryloxyaryl, alkyloxyalkyl, alkyloxyaryl or aryloxyalkyl groups; and R¹, R³, R⁴ and R⁵ may be either the same or different groups to each other.)

Further, these 2-bromo-2-fluoroketenesilylacetals are not only useful as starting materials (or synthetic intermediates) of useful compounds represented by General Formula [I] or [II], but are also useful as synthetic intermediates of other various physiologically-active substances.

As another part of the present invention, the fourth invention relates to a process for preparing the 2-bromo-2-fluoroketenesilylacetals of the third invention. Namely, the foregoing acetals represented by the following General Formula [III] are prepared by reacting dibromofluoroacetates represented by the following General Formula [VI] with halogenated silyl compounds represented by the following General Formula [VII] preferably in the presence of metal catalysts such as Zn and Cu.

CFBr₂ CO₂ R¹ General Formula [VI]

XSiR³ R⁴ R⁵ General Formula [VII]

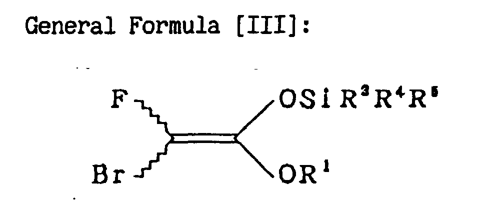

(wherein R¹s of General Formulas [VI], [VII] and [III] above are alkyl, aryl, aryloxyaryl, alkyloxyalkyl, alkyloxyaryl or aryloxyalkyl groups; R³, R⁴ and R⁵ are alkyl, aryl, alkoxy, aryloxy, aryloxyaryl, alkyloxyalkyl, alkyloxyaryl or aryloxyalkyl groups; R¹, R³, R⁴ and R⁵ may be either the same or different groups to each other and X is a halogen atom such as chlorine or bromine atom.)

In relation to the first invention of the present invention, in the β-hydroxy-α-bromo-α-fluorocarboxylates represented by General Formula [I] or [II], R¹s are preferably the same or different alkyl, aryl, aryloxyaryl, alkyloxyalkyl, alkyloxyaryl or aryloxyalkyl groups wherein the number of carbons in those groups is 1 to 10. More preferably, R¹s are lower alkyl groups, such as methyl, ethyl and t-butyl groups, and phenyl groups.

In addition, R²s are preferably alkyl, aryl, alkoxy, aryloxy, aryloxyaryl, alkyloxyalkyl, alkyloxyaryl or aryloxyalkyl groups wherein the number of carbons in those groups is 1 to 20.

In relation to the above second and third inventions of the present invention, in 2-bromo-2-fluoroketenesilylacetals represented by General Formula [III], R¹ is preferably the same or different alkyl, aryl, aryloxyaryl, alkyloxyalkyl, alkyloxyaryl or aryloxyalkyl groups wherein the number of carbons in those groups is 1 to 10. And R³, R⁴ and R⁵ are preferably the same or different alkyl, aryl, alkoxy, aryloxy, aryloxyaryl, alkyloxyalkyl, alkyloxyaryl or aryloxyalkyl groups wherein the number of carbons in those groups is 1 to 10. More preferably, R¹ is lower alkyl groups, such as methyl, ethyl and t-butyl groups, and phenyl groups, and all of R³, R⁴ and R⁵ are methyl or ethyl groups.

In aldehydes represented by General Formula [IV], R² is preferably alkyl, aryl, alkoxy, aryloxy, aryloxyaryl, alkyloxyalkyl, alkyloxyaryl or aryloxyalkyl groups wherein the number of carbons in those groups is 1 to 20. Moreover, in chiral Lewis acids represented by General Formula [V], R⁶ and R⁷ are different groups, representing alkyl and aryl groups wherein the number of carbons is 1 to 20. In particular, t-butyl group, i-propyl group and 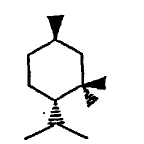 are preferable. Either R⁶ or R⁷ may be a hydrogen atom.

These chiral Lewis acids include all enantiomers. By employing either of the enantiomers, either objective compounds represented by General Formula [I] or [II] above can be obtained with high selectivity, then, a pure enantiomer can be easily separated by crystallization.

Further, R⁸ represents alkyl or aryl groups wherein the number of carbons is 1 to 50, and especially the following tosyl group (p-toluenesulfonyl group: Ts) and nitrobenzene-sulfonyl group are preferable.

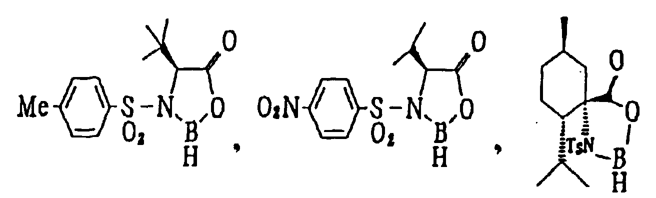

Based on selection of these Lewis acids or either enantiomer of these, the asymmetric synthesis of the objective compounds represented by General Formula [I] or [II] above can be performed with high selectivity.

In addition, the above chiral Lewis acids are known and easily prepared according to the synthetic methods described in the following literatures: for example, Org. Syn. Chem., Japan (refer to 55, 313, (1997)); Masamune et al. (J. Am. Chem. Soc., 113, 9365, (1991)); Kiyooka et al. (J. Org. Chem., 56, 2276; Tetrahedron Lett. 33, 4927, 1992); Yamamoto et al. (Synlett. 194, (1990)); and G. Helmchen (Synlett. 197, (1990)).

The present invention is described in detail along with the following synthetic process.

A process for preparing new 2-bromo-2-fluoroketenesilylacetals represented by General Formula [III] is illustrated in Scheme 1 below.

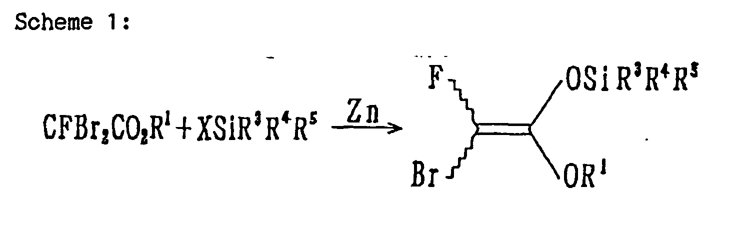

The reaction solvents used in this reaction are not limited, but ethers such as tetrahydrofuran and dimethoxyethane are especially preferable. In addition, the reaction temperature is usually in the range of -100 °C to 100°C and preferably in the range of -40°C to 20°C.

Moreover, a process for catalytic asymmetric syntheses of optically-active β-hydroxy-α-bromo-α-fluorocarboxylates represented by General Formula [I] or [II] is illustrated in Scheme 2 below.

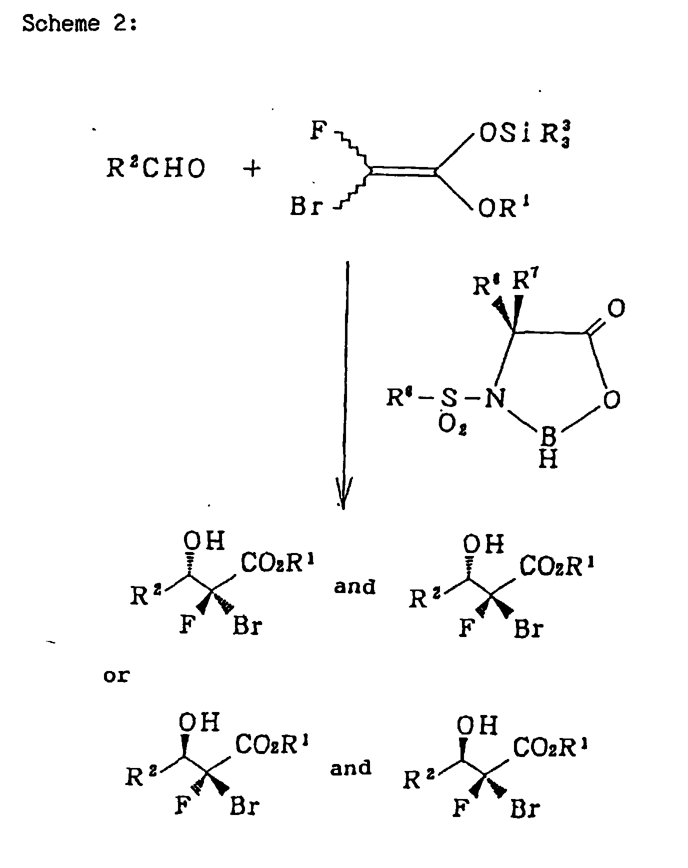

In this asymmetric synthesis, the trialkylsilyl forms are obtained as the intermediates, which are transformed into the corresponding OH group of the objective compound by hydrolysis using hydrochloric acid etc.

To obtain these optically-active β-hydroxy-α-bromo-α-fluorocarboxylates, each diastereomer is easily separated using column chromatography.

In this asymmetric synthetic process, although the reaction solvents are not specifically limited, nitroethane and propionitrile are preferable. The reaction temperature is usually in the range of -100°C to 100°C, preferably -78°C to 40 °C. The amount of Lewis acids used is preferably in the range of 0.0001 to 100 equivalent of said aldehydes, especially preferably 0.2 to 2.0 equivalents of said aldehydes.

In accordance with the present invention, since β-hydroxy-α-bromo-α-fluoro-carboxylates mentioned above have Br-atom at the 2-position (α-position), these are more reactive owing to Br-reactivity as synthetic intermediates of optically-active compounds, compared with that of all known compounds having F-atom at every 2-position as aforementioned: for example, an extension of the carbon chain is easier at the Br-position; and the Br-atom is easily converted to an H-atom by reduction, which also shows more reactivity of the compound compared with the above known compounds having F-atom at every 2-position. Accordingly, the compounds of the present invention are expected to be widely used as synthetic intermediates of optically-active compounds.

According to the inventive compounds represented by General Formula [I] or [II], such a pure enantiomer is far more effective for a synthetic intermediate to obtain an objective substance (pure enantiomeric drug) showing desirable pharmacological activities. On the other hand, if the product is a racemic form (a mixture of four compounds: the mixed compounds of General Formula [I] and [II]), it will not generally show the desirable pharmacological activities. Regarding the importance of pure enantiomeric and optically-active substances (pure enantiomers), the Thalidomide Incident is recognized as an example in which one enantiomer acted as a sedative-hypnotic drug while another one showed a teratogenic effect (refer to Encyclopedic Dictionary of Chemistry, Tokyo Kagaku Dozin, p859). Because the racemic forms of drugs are occasionally risky, it is obvious that the present invention relating to a selective synthetic process of enantiomers is required.

### INDUSTRIAL APPLICATION

With the present invention, new 2-bromo-2-fluoroketenesilylacetals are obtained by reacting dibromofluoroacetates with halogenated silyl compounds, and new optically-active β-hydroxy-α-buromo-α-fluorocarboxylates are prepared by a catalytic asymmetric synthesis of reacting the foregoing acetals with aldehydes in the presence of a catalytic amount of the chiral Lewis acids. Therefore, the foregoing carboxylates are useful as synthetic intermediates of physiologically-active compounds and can be catalytically synthesized in high asymmetric yields.

### EXAMPLES

The following examples are given to further illustrate the present invention. However, it should be understood that the present invention is not limited by these examples.

### 〈Example 1〉

### Synthesis of 1-trimethylsilyloxy-1-ethoxy-2-bromo-2-fluoroethene

After adding dibromoethane (375 µl) and trimethylsilyl chloride (450 µl) into slurry of zinc powder ( 59.68 mmol, 3.9g) in tetrahydrofuran (THF)(120 ml) at room temperature, the mixture was stirred at 40°C for 20 min.

After cooling this slurry at -20°C, trimethylsilyl chloride (62.53 mmol, 7.94 ml) was again added and ethyl dibromo-fluoroacetate (56.84 mmol, 15.0 g) was dropped into the mixture.

After stirring at the same temperature for 1 hr, anhydrous pentane (200 ml) was added, and the resulting insoluble substance was filtered and the filtrate was concentrated under reduced pressure. This treatment was repeated once more and 7.7g (53% yield) of the objective compound was obtained by vacuum distillation. The analytical data of this product was as follows:
colorless oil: the major (E-form)/the minor (Z-form) = 61.6/38.4
   - ¹H-NMR (CDCl₃):: the major (E-form); δ 0.25 (s, 9H),
   1.26 (t, J=7.1 Hz, 3H),
   3.88 (q, J=7.1 Hz, 2H),
   the minor (Z-form); δ 0.25 (s, 9H),
   1.28 (t, J=7.1 Hz, 3H),
   3.95 (q, J=7.1 Hz, 2H);
   - ¹⁹F-NMR (CDCl₃):: the major (E-form); δ -133.14 (s, 1F),
   the minor (Z-form); δ -134.52 (s, 1F)
b. p. 37.0-38.4 °C/ 1.2 mmHg

### The structural determination of the products E-form and Z-form

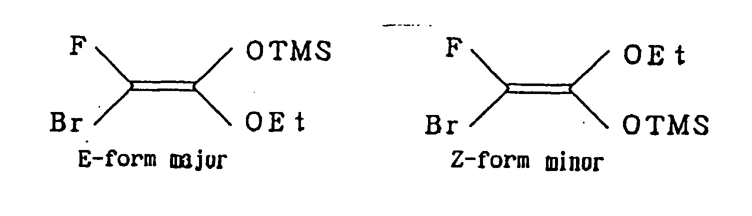

Each methylene signal in the ethyl group by the ¹H-NMR spectrum was assigned to the corresponding major and minor compounds. Subsequently, although the ¹⁹F-NOE measurement on detection difference based on the ¹H irradiation was conducted with irradiating the methylene signal of the major form, no NOE was observed. In addition, when a similar measurement was conducted by irradiating the methylene signal of the minor form, the ¹⁹F-related-NOE signal of the minor form was observed. From these data, the chemical structures of the major and minor forms were determined as above, respectively (herein, TMS represents a trimethylsilyl group).

### 〈Example 2〉

### Catalytic asymmetric synthesis of (+)-(2S, 3R)-erythro-ethyl-2-bromo-2-fluoro-3-hydroxy-3-phenylpropanoate and (-)-threo-ethyl-2-bromo-2-fluoro-3-hydroxy-3-phenylpropanoate

To a solution of the following sulfonamide (0.2 mmol, 71 mg) in nitroethane (3 ml), 1.0 M solution of BH₃-THF (0.2 mmol, 200 µl) was added at 25°C. The mixture was stirred at the same temperature for 10 min, further stirred at 45°C for 1 hr and the following chiral Lewis acid was obtained.

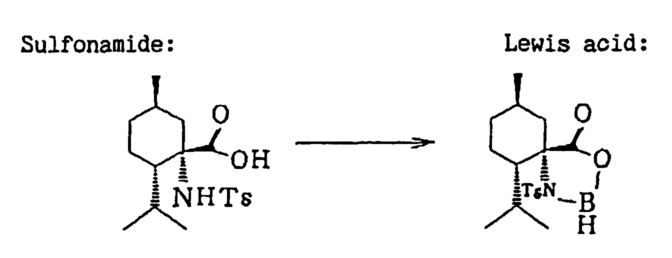

To this reaction solution cooled at -78°C, 1-trimethylsilyloxy-1-ethoxy-2-bromo-2-fluoroethene (1.2 mmol, 309 mg) was added and subsequently a solution of benzaldehyde (1.0 mmol, 102 µl) in nitroethane (2 ml) was dropped over 3 hr.

After further stirring at the same temperature for an hour, the reaction solution was mixed with diethyl ether (50 ml) and the mixture was subjected to extraction with aqueous saturated sodium bicarbonate (10 ml) and saturated brine (10 ml), dried, and concentrated under reduced pressure.

After stirring the crude product diluted with THF (10 ml) and 2 N-hydrochloric acid (2 ml) for 1 hr, the mixture diluted with diethyl ether (50 ml) was extracted with saturated sodium bicarbonate (10 ml) and saturated brine (10 ml), dried, and concentrated under reduced pressure.

The crude product obtained was purified by silica gel chromatography (eluent: hexane/ethyl acetate=12/1), and the product as illustrated above was obtained in yields of 178.3 mg (62% yield) of the erythro form and 80.9 mg (28% yield) of the threo form. The optical yields of these products corresponding to the General Formula [II] as aforementioned showed high enantio selectivity of 98% ee as an erythro form and of 90% ee as a threo form by analysis using Daisel Chiracel OB-H (eluent: hexane/ethanol=20/1) and Daisel Chiracel OD-H (eluent: hexane/ethanol=20/1).

Terminology of erythro and threo forms is defined with the relative position between the OH and F groups, wherein an erythro form represents a form where two groups are positioned on one side, whereas a threo form represents another form where two groups are positioned on the opposite side (and so forth). These analytical data are as follows:
The erythro form; colorless oil
   - ¹H-NMR(CDCl₃):: δ 1.24 (t, J=7.1 Hz, 3H),
   3.03-3.28 (brs, 1H),
   4.25 (q, J=7.1 Hz, 2H),
   5.24 (d, J=17.8 Hz, 1H),
   7.30-7.52 (m, 5H)
   - ¹⁹F-NMR (CDCl₃):: δ -131.65 (d, J=14.8 Hz, 1F)
[α]_{D}²⁴ +1.62 (CHCl₃, c=1.23)
The threo form; colorless oil
   - ¹H-NMR(CDCl₃):: δ 1.37 (t, J=7.1 Hz, 3H),
   2.80-3.00 (brs, 1H), 4.40 (q, J=7.1 Hz, 2H),
   5.30 (d, J=20.7 Hz, 1H),
   7.33-7.58 (m, 5H)
   - ¹⁹F-NMR(CDCl₃):: δ -137.23 (d, J=20.7 Hz, 1F)
[α]_{D}²⁴ -57.78 (CHCl3, c=0.93)

### Absolute structure of the product

Pyridine (1.16 mmol, 95 µl), (1S)-(-)-camphanic chloride (0.581 mmol, 126 mg) and dimethylaminopyridine (2 mg) were added to a solution of the erythro form (0.116 mmol, 33.8 mg) in dichloromethane at 25 °C and stirred for 19 hr. The reaction solution mixed with diethyl ether (30 ml) was extracted with 2N-hydrochloric acid (5 ml) and aqueous saturared sodium bicarbonate (5 ml), dried, and concentrated under reduced pressure.

The crude product obtained was purified by silica gel chromatography (eluent: hexane/ethyl acetate=5/1) to give 53.6 mg (98% yield) of the objective compound. Further, a crystal sample of the compound was recrystalized with ether-hexane, and the erythro form was decided to be the absolute configuration of (2S, 3R) by X-ray crystal analysis.

The analytical data of the erythro form are as follows:
White crystals; m. p. 117.6-118.4°C
   - ¹H-NMR(CDCl₃):: δ 0.97 (s, 3H), 1.13 (s, 3H),
   1.14 (s, 3H), 1.23 (t, J=7.1 Hz, 3H),
   1.65-1.81 (m, 14H), 1.90-2.20 (m, 1H),
   2.49-2.66 (m, 1H), 4.23 (q, J=7.1 Hz, 2H),
   6.52 (d, J=22.5 Hz, 1H), 7.30-7.52 (m, 5H)
   - ¹⁹F-NMR(CDCl₃):: δ -131.37 (d, J=22.5 Hz, 1F)

Similar asymmetric syntheses of various aldehydes in addition to benzaldehyde in Example 2 were performed, and the analytical data of the products obtained are mentioned below. In addition, the yield of each Example is illustrated in the following Table 1. These products which correspond to the above General Formula [II] showed high enantio selectivity.

### 〈Example 3〉

### (+)-erythro-ethyl-2-bromo-2-fluoro-3-hydroxy-5-phenyl-4-pentenoate and (+)-threo-ethyl-2-bromo-2-fluoro-3-hydroxy-5-phenyl-4-pentenoate

The erythro form: colorless oil
   - ¹H-NMR(CDCl₃):: δ 1.34 (t, J=7.2 Hz, 3H),
   1.58 (brs, 1H),
   4.36 (q, J=7.2 Hz, 2H),
   4.75-4.89 (m, 1H),
   6.24 (ddd, J=1.0, 7.0, 15.9 Hz, 1H),
   6.8 (d, J=6.8 Hz, 1H),
   7.25-7.43 (m, 5H)
   - ¹⁹F-NMR(CDCl ):: δ -130.10 (d, J=15.2 Hz, 1F)
[α]_{D}²⁴ +22.86 (CHCl₃, c=0.82 )
The threo form: colorless oil
   - ¹H-NMR(CDCl₃):: δ 1.39 (t, J=7.1 Hz, 3H),
   2.60 (m, 1H),
   4.41 (q, J=7.1 Hz, 2H),
   4.80-5.00 (m, 1H),
   6.39 (dd, J=6.7, 15.6 Hz, 1H),
   6.87 (d, J=15.6 Hz, 1H),
   7.27-7.50 (m, 5H)
   - ¹⁹F-NMR(CDCl₃):: δ -137.02 (d, J=19.4 Hz, 1F)
[α]_{D}²⁴ +27.41 (CHCl₃, c=0.85)

### 〈Example 4〉

### (+)-erythro-ethyl-2-bromo-2-fluoro-3-hydroxy-5-phenylpentanoate and (+)-threo-ethyl-2-bromo-2-fluoro-3-hydroxy-5-phenylpentanoate

The erythro form: colorless oil
   - ¹H-NMR(CDCl₃):: δ 1.32 (t, J=7.1 Hz, 3H),
   1.80-2.12 (m, 2H),
   2.63-3.06 (m, 3H),
   3.91-4.09 (m, 1H),
   4.33 (q, J=7.1 Hz, 2H),
   7.13-7.40 (m, 5H)
   - ¹⁹F-NMR(CDCl₃):: δ -129.13 (d, J=12.3 Hz, 1F)
[α]_{D}²⁴ +32.66 (CHCl₃, c=0.82)
The threo form: colorless oil
   - ¹H-NMR(CDCl₃):: δ 1.36 (t, J=7.1 Hz, 3H),
   1.80-2.03 (m, 1H),
   2.30-2.50 (m, 2H),
   2.67-3.08 (m, 2H),
   4.06-4.29 (m, 1H),
   4.37 (q, J=7.1 Hz, 2H),
   7.12-7.38 (m, 5H)
   - ¹⁹F-NMR(CDCl₃):: δ -136.85 (d, J=19.9 Hz, 1F)
[α]_{D}²⁴ +33.23 (CHCl₃, c=1.01)

### 〈Example 5〉

### (-)-erythro-ethyl-2-bromo-2-fluoro-3-acetoxy-4-benzyloxybutanoate and (+)-threo-ethyl-2-bromo-2-fluoro-3-acetoxy-4-benzyloxybutanoate

The erythro form: colorless oil
   - ¹H-NMR(CDCl₃):: δ 1.24 (t, J=7.1 Hz, 3H),
   2.20 (s, 3H),
   3.67 (dd, J=1.0, 6.9 Hz, 2H),
   4.05-4.28 (m, 2H),
   4.41-4.45 (m, 2H),
   5.80 (dt, J=7.0, 22.4 Hz, 1H),
   7.21-7.45 (m, 5H)
   - ¹⁹F-NMR(CDCl₃):: δ -131.41 (d, J=22.2 Hz, 1F)
[α]_{D}²⁴ -0.22 (CHCl₃, c=0.89)
The threo form: colorless oil
   - ¹H-NMR(CDCl₃):: δ 1.32 (t, J=7.1 Hz, 3H),
   2.10 (s, 3H),
   3.84 (ddd, J=1.0, 7.8, 11.4 Hz, 1H),
   4.16 (dd, J=3.1, 11.4 Hz, 1H),
   4.26-4.38 (m, 2H),
   4.49-4.69 (m, 2H),
   5.86 (ddd, J=3.1, 7.8, 22.1 Hz, 1H),
   7.25-7.40 (m, 5H)
   - ¹⁹F-NMR(CDCl₃):: δ -131.40 (d, J=22.2 Hz, 1F)
[α]_{D}²⁴ +15.31 (CHCl₃, c=0.83)

### 〈Example 6〉

### (+)-erythro-ethyl-2-bromo-2-fluoro-3-hydroxy-3-cyclohexylpropanoate and (+)-threo-ethyl-2-bromo-2-fluoro-3-hydroxy-3-cyclohexylpropanoate

The erythro form: colorless oil
   - ¹H-NMR(CDCl₃):: δ 1.12-2.03 (m, 11H),
   1.37 (t, J=7.1 Hz, 3H),
   2.45 (brs, 1H),
   3.84-4.03 (m, 1H),
   4.38 (q, J=7.1 Hz, 2H)
   - ¹⁹F-NMR(CDCl₃):: δ -128.70 (d, J=21.8 Hz, 1F)
[α]_{D}²⁴ +5.54 (CHCl₃, c=0.76)
The threo form: colorless oil
   - ¹H-NMR(CDCl₃):: δ 1.07-2.41 (m, 12H),
   1.37 (t, J=7.2 Hz, 3H),
   3.91-4.13 (m, 1H),
   4.37 (q, J=7.2 Hz, 2H)
   - ¹⁹F-NMR(CDCl₃):: δ -133.98 (dd, J=2.4, 23.1 Hz, 1F)
[α]_{D}²⁴ +11.41 (CHCl₃, c=0.88)

### 〈Example 7〉

### (+)-erythro-ethyl-2-bromo-2-fluoro-3-hydroxyhexanoate and (+)-threo-ethyl-2-bromo-2-fluoro-3-hydroxyhexanoate

The erythro form: colorless oil
   - ¹H-NMR(CDCl₃):: δ 0.96 (t, J=7.1 Hz, 3H),
   1.38 (t, J=7.1 Hz, 3H),
   1.32-1.78 (m, 4H),
   2.64 (brs, 1H),
   3.98-4.13 (m, 1H),
   4.38 (q, J=7.1 Hz, 2H)
   - ¹⁹F-NMR(CDCl₃):: δ -129.63 (d, J=13.0 Hz, 1F)
[α]_{D}²⁴ +16.36 (CHCl₃, c=1.02)
The threo form: colorless oil
   - ¹H-NMR(CDCl₃):: δ 0.99 (t, J=7.4 Hz, 3H),
   1.36 (t, J=7.1 Hz, 3H),
   1.42-2.23 (m, 5H),
   4.06-4.29 (m, 1H),
   4.37 (q, J=7.1 Hz, 2H)
   - ¹⁹F-NMR(CDCl₃):: δ -137.39 (d, J=20.5 Hz, 1F)
[α]_{D}²⁴ +20.58 (CHCl₃, c=1.01).

### 〈Example 8〉

### (+)-erythro-ethyl-2-bromo-2-fluoro-3-hydroxy-5-methylhexanoate and (+)-threo-ethyl-2-bromo-2-fluoro-3-hydroxy-5-methylhexanoate

The erythro form: colorless oil
   - ¹H-NMR(CDCl₃):: δ 0.92 (t, J=6.5 Hz, 3H),
   0.97 (d, J=6.7 Hz, 3H),
   1.19-1.30 (m, 1H),
   1.36 (t, J=7.2 Hz, 3H),
   1.58-2.02 (m, 2H),
   2.55-2.62 (m, 1H),
   4.02-4.20 (m, 1H),
   4.29-4.43 (m, 2H)
   - ¹⁹F-NMR(CDCl₃):: δ -129.86 (d, J=15.2 Hz, 1F)
[α]_{D}²⁴ +24.38 (CHCl₃, c=0.66)
The threo form: colorless oil
   - ¹H-NMR(CDCl₃):: δ 0.99 (d, J=6.2 Hz, 3H),
   1.02 (d, J=6.4 Hz, 3H),
   1.38 (t, J=7.2 Hz, 3H) ,
   1.48-2.25 (m, 4H),
   4.12-4.32 (m, 1H),
   4.39 (q, J=7.2 Hz, 2H)
   - ¹⁹F-NMR(CDCl₃):: δ -137.67 (d, J=20.5 Hz, 1F)
[α]_{D}²⁴ +28.21 (CHCl₃, c=0.91)

### 〈Example 9〉

### (+)-erythro-ethyl-2-bromo-2-fluoro-3-hydroxy-4-ethylhexanoate and (+)-threo-ethyl-2-bromo-2-fluoro-3-hydroxy-4-ethylhexanoate

The erythro form: colorless oil
   - ¹H-NMR(CDCl₃):: δ 0.80-0.98 (m, 6H),
   1.20-1.73 (m, 5H),
   1.36 (t, J=7.2 Hz, 3H),
   2.38 (brs, 1H),
   4.06-4.25 (m, 1H),
   4.36 (q, J=7.2 Hz, 2H)
   - ¹⁹F-NMR(CDCl₃):: δ -128.79 (d, J=23.7 Hz, 1F)
[α]_{D}²⁴ +19.20 (CHCl₃, c=0.67)
The threo form: colorless oil
   - ¹H-NMR(CDCl₃):: δ 0.97 (t, J=7.7 Hz, 6H),
   1.02-2.35 (m, 6H),
   1.37 (t, J=7.1 Hz, 3H),
   4.17-4.30 (m, 1H),
   4.37 (q, J=7.1 Hz, 2H)
   - ¹⁹F-NMR(CDCl₃):: δ -136.05 (d, J=25.0 Hz, 1F)
[α]_{D}²⁴ +15.58 (CHCl₃, c=0.97)

**Table 1**

| Example | Aldehyde | yield (%) | erythro /threo | ee(erythro) (%) | ee(threo) (%) |
|---|---|---|---|---|---|
| 2 | PhCHO | 90 | 69/31 | 98 | 90 |
| 3 | (E)-PhCH=CHCHO | 96 | 57/43 | 83 | 83 |
| 4 | C₆H₅CH₂CH₂CHO | 89 | 46/54 | 98 | 98 |
| 5 | BnOCH₂CHO ^{b)} | 81 ^{a)} | 57/43 | 97 | 97 |
| 6 | C₆H₁₁ CHO | 74 | 52/48 | 94 | 89 |
| 7 | C₃H₇CHO | 90 | 46/54 | 97 | 98 |
| 8 | Me₂(CH₂)₂CHO | 96 | 48/52 | 98 | 98 |
| 9 | Et₂CHCHO | 70 | 54/46 | 99 | 98 |

| | | | | | |
|---|---|---|---|---|---|
| a) Determined by isolation yield of the corresponding acetate | | | | | |
| b) Bn: benzyl group | | | | | |

### 〈Example 10〉

### Synthesis of 1-trimethylsilyloxy-1-isopropoxy-2-bromo-2-fluoroethene

Isopropyl dibromofluoroacetate instead of ethyl dibromofluoroacetate was used under similar conditions mentioned in Example 1 and the product showing the following analytical data was obtained:
Colorless oil; major/minor=62.1/37.9
   - ¹H-NMR(CDCl₃):: δ 0.22-0.28 (m, 9H),
   1.19-1.27 (m, 6H),
   4.20-4.37 (m, 1H)
   - ¹⁹F-NMR(CDCl₃):: major δ -131.82 (s, 1F),
   minor δ -138.08 (s, 1F)
b. p. 55-62 °C/7 mmHg

### 〈Example 11〉

### Catalytic asymmetric syntheses of (-)-Erythro-isopropyl-2-bromo-2-fluoro-3-hydroxy-5-phenyl-4-pentenoate and (-)-threo-isopropyl-2-bromo-2-fluoro-3-hydroxy-5-phenyl-4-pentenoate

1-trimethylsilyloxy-1-isopropoxy-2-bromo-2-fluoroethene instead of 1-trimethyl silyloxy-1-ethoxy-2-bromo-2-fluoroethene was used under similar conditions mentioned in Example 2 and the product showing the following analytical data was obtained:
The erythro form; colorless oil
   - ¹H-NMR(CDCl₃):: δ 1.29 (d, J=6.3 Hz, 3H),
   1.30 (d, J=6.3 Hz, 3H),
   2.20 (s, 3H),
   5.15 (qq, J=6.3, 6.3 Hz, 1H),
   6.05 (dd, J=23.1 , 8.3 Hz, 1H),
   6.12 (dd, J=15.5, 8.6 Hz, 1H),
   6.80 (d, J=15.5 Hz, 1H),
   7.22-7.42 (m, 5H)
   - ¹⁹F-NMR(CDCl₃):: δ -130.14 (d, J=23.1 Hz, 1F)
[α]_{D}²² -15.83 (CHCl₃, c=0.619)
The threo form: white crystals
   - ¹H-NMR(CDCl₃):: δ 1.29 (d, J=6.3 Hz, 3H),
   1.34 (d, J=6.3 Hz, 3H),
   2.06 (s, 3H),
   5.18 (qq, J=6.3, 6.3 Hz, 1H),
   6.03 (dd, J=8.4, 22.8 Hz, 1H),
   6.25 (dd, J=8.4, 15.9 Hz, 1H),
   6.92 (d, J=15.9 Hz, 1H),
   7.28-7.51 (m, 5H)
   - ¹⁹F-NMR(CDCl₃):: δ -134.6 (d, J=22.8 Hz, 1F)
[α]_{D}²² -28.813 (CHCl₃, c=0.472).

The following Table 2 shows the yields of the above products.

**Table2**

| Aldehyde | yield (%) | erythro /threo | ee(erythro) (%) | ee(threo) (%) |
|---|---|---|---|---|
| (E)-phCH=CHCHO | 89 | 39/61 | 95 | 95 |

## Claims

1. Optically-active β-hydroxy-α-bromo-α-fluorocarboxylates represented by the following General Formula [I] or [II], 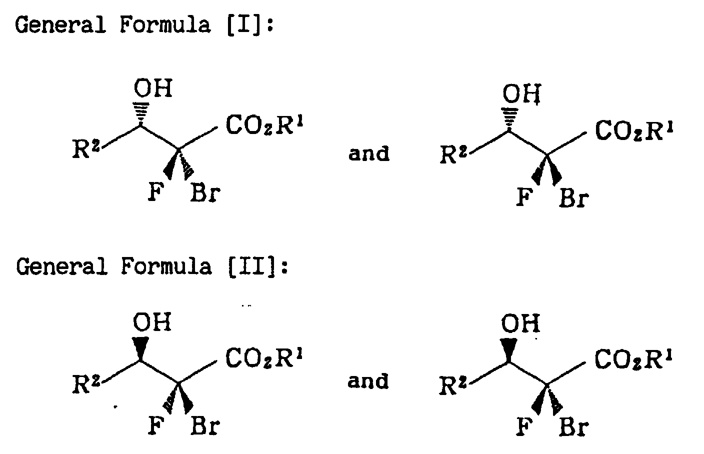 (wherein R¹s of General Formulas [I] and [II] are alkyl, aryl, aryloxyaryl, alkyloxyalkyl, alkyloxyaryl or aryloxyalkyl groups; R²s are alkyl, aryl, alkoxy, aryloxy, aryloxyaryl, alkyloxyalkyl, alkyloxyaryl or aryloxyalkyl groups; R¹ and R² may be either the same or different groups to each other).

2. The optically-active β-hydroxy-α-bromo-α-fluorocarboxylates as claimed in claim1 wherein the number of carbons in said R¹ is 1 to 10 and the number of carbons in said R² is 1 to 20.

3. A process for catalytic asymmetric synthesis of the optically-active β-hydroxy-α-bromo-α-fluorocarboxylates represented by the following General Formula [I] or [II], wherein said carboxylates are asymmetrically prepared by reacting 2-bromo-2-fluoroketenesilylacetals represented by the following General Formula [III] with aldehydes represented by the following General Formula [IV] in the presence of chiral Lewis acids represented by the following General Formula [V], 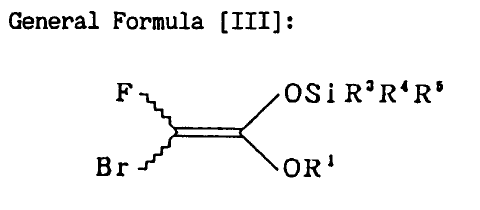
R²CHO General Formula [IV] 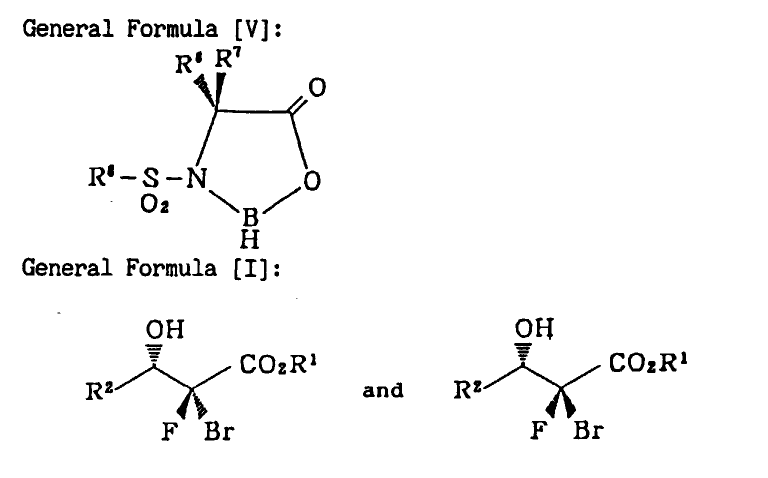 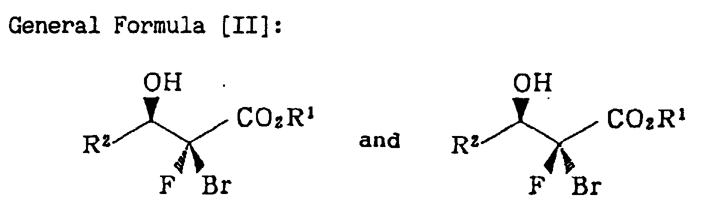
[wherein R¹s of General Formulas [I], [II], [III], [IV] and [V] are alkyl, aryloxy, aryloxyaryl, alkyloxyalkyl, alkyloxyaryl or aryloxyalkyl groups; R² , R³ , R⁴, R⁵, R⁶, R⁷ and R⁸ are alkyl, aryl, alkoxy, aryloxy, aryloxyaryl, alkyloxyalkyl, alkyloxyaryl or aryloxyalkyl groups; R¹, R² , R³ , R⁴, R⁵, R⁶, R⁷ and R⁸ may be either the same or different groups to each other (herein, R⁶ and R⁷ are not the same as each other but either R⁶ or R⁷ may be a hydrogen atom)].

4. A process for catalytic asymmetric synthesis of the optically-active β-hydroxy-α-bromo-α-fluorocarboxylates as claimed in claim 3 wherein the number of carbons in said R¹, R³, R⁴ and R⁵ is 1 to 10; the number of carbons in said R² is 1 to 20; the number of carbons in said R⁶ and R⁷ is 1 to 20; the number of carbons in said R⁸ is 1 to 50.

5. A process for catalytic asymmetric synthesis of the optically-active β-hydroxy-α-bromo-α-fluorocarboxylates as claimed in claim 3 wherein a catalytic amount of said chiral Lewis acids is used.

6. A process for catalytic asymmetric synthesis of the optically-active β-hydroxy-α-bromo-α-fluorocarboxylates as claimed in claim 5 wherein the amount of said Lewis acids used is in the range of 0.0001 to 100 equivalents of said aldehydes.

7. A process for catalytic asymmetric synthesis of the optically-active β-hydroxy-α-bromo-α-fluorocarboxylates as claimed in claim 3 wherein a reaction solvent is nitroethane or propionitrile to perform the synthesis within the range of -100°C to 100°C.

8. 2-bromo-2-fluoroketenesilylacetals represented by the following General Formula [III], 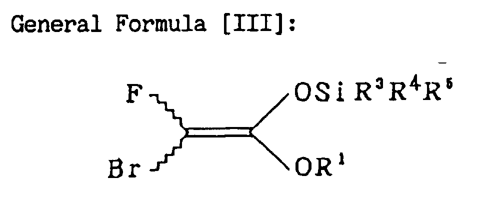 (wherein R¹ of General Formula [III] is alkyl, aryl, aryloxyaryl, alkyloxyalkyl, alkyloxyaryl or aryloxyalkyl groups; R³, R⁴ and R⁵ are alkyl, aryl, alkoxy, aryloxy, aryloxyaryl, alkyloxyalkyl, alkyloxyaryl or aryloxyalkyl groups; R¹, R³, R⁴ and R⁵ may be either the same or different groups to each other).

9. 2-bromo-2-fluoroketenesilylacetals as claimed in claim 8 wherein the number of carbons in said R¹, R³, R⁴ and R⁵ is 1 to 10.

10. A process for preparing 2-bromo-2-fluoroketenesilylacetals represented by the following General Formula [III] wherein said acetals are obtained by reacting dibromofluoroacetates represented by the following General Formula [VI] With halogenated silyl compounds represented by the following General Formula [VII],
CFBr₂ CO₂ R¹ General Formula [VI]
XSiR³ R⁴ R⁵ General Formula [VII] 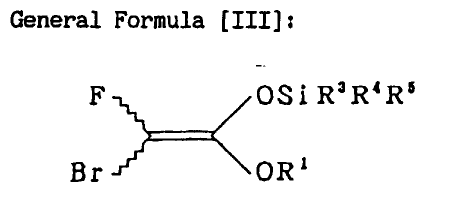
(wherein R¹s of General Formulas [VI], [VII] and [III] are alkyl, aryl, aryloxyaryl, alkyloxyalkyl, alkyloxyaryl or aryloxyalkyl groups; R³, R⁴ and R⁵ are alkyl, aryl, alkoxy, aryloxy, aryloxyaryl, alkyloxyalkyl, alkyloxyaryl or aryloxyalkyl groups; R¹, R³, R⁴ and R⁵ may be either the same or different groups to each other; and X is a halogen atom).

11. A process for preparing 2-bromo-2-fluoroketenesilylacetals as claimed in claim 10 wherein the number of carbons in said R¹, R³, R⁴ and R⁵ is 1 to 10 and X is chlorine or bromine atom.

12. A process for preparing 2-bromo-2-fluoroketenesilylacetals as claimed in claim 10 to perform the reaction in the presence of metal catalysts.

13. A process for preparing 2-bromo-2-fluoroketenesilylacetals as claimed in claim10 wherein the reaction solvent is ether-solvents to perform the reaction within the range of -100°C to 100°C.
